# EUROPEAN PATENT APPLICATION

(11) **EP 1 764 135 A1**
(43) Date of publication of application: **21.03.2007**
(21) Application number: 06019246.5
(22) Date of filing: 14.09.2006
(51) Int. Cl.: A61Q 19/00, A61K 8/33, A61K 8/34, A61K 8/58, A61K 8/81

(54) **Cold production method for pearly lustre preparations containing alcohols**

(30) Priority: 19.09.2005 US 229826
(71) Applicant: CLARIANT INTERNATIONAL LTD., 4132 Muttenz (CH)
(72) Inventor: Porter, Sandra Nicholson, Huntersville NC 28078 (US)
(74) Representative: Paczkowski, Marcus

(57) **Abstract**

Compositions and methods are disclosed for producing pearlized compositions of alcoholic hydrogels at ambient temperatures. More particularly, compositions and methods are disclosed for pearlized alcoholic hydrogel compositions with stearyl alcohol at preparation temperatures below the melting point of stearyl alcohol, which is solid at room temperature. Pearlizing effects are achieved in alcoholic hydrogels at low temperatures by the addition to the alcoholic hydrogel of stearyl alcohol in a siloxane form as stearyl alcohol trialkylsiloxane. By incorporating the siloxane form of stearyl alcohol, which previously could be only handled at anhydrous conditions, into the hydroalcoholic gel, one obtained a stable pearlized alcoholic hydrogel having a lustrous pearlescent appearance. Preparation was accomplished without heating above room temperature, without a pearlescent wax, and without a potentially skin irritating surfactant.

## Description

This invention concerns generally with pearlescent cosmetic and pharmaceutical compositions, wherein such compositions comprise alcohol and more particularly relates to a process for the production of such preparations without heating.

The first pearlescence used in cosmetics in the middle ages, was a pearlizing paste of natural fish scales. At the beginning of the present century, it was discovered that bismuth oxide chlorides were also capable of producing pearlescence. In modern cosmetics, pearlizing waxes, particularly of the glycol monofatty acid ester and difatty acid ester type, found importance and are widely used for the production of a pearlescence appearance in hair shampoos and shower gels, which generally have a low alcoholic content. Commercially available pearlizing waxes have melting points above 50°C and, accordingly, cannot simply be incorporated into cold water-based formulations. Because of this limitation, a hot process is generally employed in which the waxes are melted and then allowed to crystallize out slowly in the formulation. The pearlizing effect and the degree of brilliance of the pearlescence of the final product are dependent upon the particle fineness of the wax crystals formed during the cooling step.

In US Patent No. 4,007, 261, pearlescent compositions are disclosed which consist essentially of aqueous emulsions or dispersions of alkyldimethylamine oxides having from 16 to 22 carbons in the alkyl chain. The alkyl is preferably straight chain, and if branching is present, branching should be minimized. All of the preparations are heated with agitation to provide thorough mixing followed by gradual cooling with moderate agitation. Furthermore, US Patent No. 4,007, 261 discloses that substances such as stearyl alcohol which is typically added to hair conditioning agents to provide a pearlescent effect is not required. Stearyl alcohol, which has a melting point of 59°C, is a white solid at room temperature. Stearyl alcohol is soluble in alcohol, but is insoluble in water. Incorporation of stearyl alcohol into water based cosmetics, such as alcoholic hydrogels is limited to low concentration levels because of the limited solubility of stearyl alcohol in such alcoholic hydrogels.

In US Patent No. 4,275,055, a hair conditioner is disclosed in which a stable pearlescent effect is achieved in the absence of nacreous or synthetic pearlizing agents, but in the presence of two conditioning agents which together produce a pearlescent effect. The conditioning agents are stearamidopropyl dimethyl benzylammonium chloride and stearyl dimethyl benzylammonium chloride, being present in specific ratios. The formulation of the composition required heating the components to a temperature between 60 and 80°C, followed by slow cooling until the pearlescence appears.

US patent No. 4,777,038 discloses a free-flowing aqueous concentrate for imparting a pearlescent appearance to cosmetics and surfactants. The free-flowing aqueous concentrate is prepared by heating the fatty components such as ethylene glycol stearate and triethylene glycol distearate beyond their melting points and mixing the components at a temperature of 75 to 100°C, followed by gradual cooling with mixing. The thus prepared concentrates produce pearlescence in aqueous cationic or anionic surfactant preparations by dispersing the concentrate into the surfactant preparation with gentle stirring.

US Patent 6,610,315 discloses a composition for topical application of a stable hydroalcoholic composition for maintaining or improving skin conditions. The composition comprises a lower alcohol (C1 to C4 alcohol) and water in a ratio of from about 35:65 to 100:0 and a thickener comprised of between 0.5 to 8.0 percent of at least two emulsifiers. The composition optionally includes other ingredients such as antimicrobial agents and emollients.

The high alcoholic content of typical hydroalcoholic compositions, or hydrogels, prevents the heating of these hydrogels to permit the incorporation ofpearlescent wax into such alcoholic hydrogels, because either conventional pearlescent wax must be heated to a temperature above the vaporization temperature of the alcoholic hydrogel, or the alcoholic hydrogel would have to be heated above its vaporization temperature in order to combine or disperse the pearlescent wax. In either case, heating the alcoholic hydrogel would result in the release of alcohol from the gel and create safety and production problems.

One attempt to solve the problem of providing alcoholic hydrogel with a pearlescent appearance can be found in US Patent 6,610,315. US Patent 6,610,315 discloses a composition for topical application of a stable hydroalcoholic composition for maintaining or improving skin conditions. The composition comprises a lower alcohol (C₁ to C₄ alcohol) and water in a ratio of from about 35:65 to 100:0 and a thickener comprised of between 0.5 to 8.0 percent of at least two emulsifiers. The composition optionally includes other ingredients such as antimicrobial agents and emollients. The ethanol was heated to disperse the pearlescent wax in a sealed contained to obtain the pearlescent appearance.

Emulsifiers are typically included of a wide class of materials referred to as surfactants. Although the use of surfactants in cosmetic and pharmaceutical preparations, including surfactants such as ionic, nonionic, amphoteric, zwitterionic surfactants, may provide an effective method for suspending a pearlizing wax in an alcoholic hydrogel, but the presence of these surfactants in the cosmetic or pharmaceutical composition may be irritating to human skin or their composition may interfere with the gelling capacity of suitable hydroalcoholic polymeric thickeners.

More recently, US Patent No. 6,727,217 discloses pearlescent surfactant compositions such as shampoos which are prepared at a temperature of from 10°C to 45°C by providing an aqueous surfactant solution and then contacting the aqueous surfactant solution with a composition comprised of a pearlizing wax and a polyol ester.

Pearlescent cosmetic and pharmaceutical compositions and pearlizing concentrate compositions, particularly for those compositions comprising alcoholic hydrogels having a high alcoholic content, are sought which can be prepared without heating above room temperature conditions, and wherein the cosmetic and pharmaceutical compositions and pearlizing concentrate compositions are essentially surfactant free.

Applicant surprisingly discovered that at ambient conditions, stearyl alcohol can be incorporated into alcoholic hydrogels at relatively high concentrations and subsequently produce a pearlescent appearance in the alcoholic hydrogel, without the need for heating the alcohol or without the need for adding a surfactant. Furthermore, the pearlescent appearance is achieved without heating a pearlescent wax. Applicant discovered that a silicone form of stearyl alcohol can be combined with an alcoholic hydrogel at ambient conditions (10 to 30°C) and after such combination, the stearyl alcohol, which is essentially insoluble in the alcoholic hydrogel is released in pure form into the hydrogel, producing a lustrous pearlescent appearance. In addition, the presence of the stearyl alcohol in the alcoholic hydrogel imparts a soothing and smooth feel to human skin. Still further, the alcoholic hydrogels of the present invention can be employed alone or in combination with active antibacterial agents in antibacterial preparations such as hand sanitizers and hand lotions. Applicant further discovered that the observed brilliant pearlescent appearance can be controlled to provide varying degrees of opacity from a metallic pearlescence to opaque.

In one embodiment, the present invention is a pearlescent alcoholic hydrogel of stearyl alcohol, wherein the alcoholic hydrogel has a low molecular weight alcoholic content of from 0.001 to 80 weight percent and is essentially free of pearlescent wax. Wherein essentially free of a pearlescent wax means that the composition of the present invention contains less than 0.05 weight percent of a conventional fatty acid pearlescent wax. In another embodiment, the present invention is a method for preparing a pearlescent alcoholic hydrogel of stearyl alcohol at ambient conditions.

The pearlescent compositions of the present invention comprise an alcoholic hydrogel comprising a low molecular weight monoalcohol having from 2 to 4 carbon atoms per molecule, a thickener, and siloxane compound. The pearlescent compositions of the present invention are essentially free of traditional fatty acid pearlescent wax. Examples of such pearlescent waxes are fatty acid monoalkanolamides, fatty acid dialkanolamides, monoesters or diesters of ethylene glycol or mixtures thereof, propylene glycol or its oligomers, mono- or diesters of alkylene glycols with fatty acids, fatty acids and their metal salts, monoesters or polyesters of glycerol with carboxylic acids and keto sulfones of various types. By the term essentially free of pearlescent wax, it is meant that the finished composition of the present invention contains less than 0.05 weight percent of pearlescent wax.

The thickener of the present invention functions as the gelling agent which when combined with the alcohol provides the hydroalcoholic gel. The thickener may be selected both as to type and quantity to give products of various viscosities. In the preferred form of this invention, the thickener is selected so as to produce an elegantly formed and stable gel which is either semi-solid or pourable. A variety of thickeners may be used for the present purposes. Any suitable thickener which is capable of suspending particulates in a hydroalcoholic gel may be employed. Preferred thickeners include carbomers, crosslinked sulfonic acid copolymers and mixtures thereof. Carbomers include addition polymers of acrylic acid crosslinked with an unsaturated polyfunctional agent such as a polyallyl ether of sucrose. Such carbomer polymers are described in U.S. Pat. Nos. 2,798,053 and 3,133,865, have the CTFA (Cosmetic, Toiletry and Fragrance Association) adopted name of "Carbomer" and are commercially available under the tradenames CARBOPOL(R) 934, 940 and 941 from Noveon. Carbomers must be neutralized with an effective amount of an alkaline material in order to form a gel. Suitable neutralizing agents for carbomers incude organic amines, such as triethanolamine, triethylamine, isopropylamine, diisopropylamine, and the like. Inorganic bases such as sodium hydroxide, potassium hydroxide, Ca(OH)₂, and the like can also be use to neutralize carbomers to form gels.

Crosslinked sulfonic acid copolymers include copolymers of the ammonium salt of 2-acrylamido-2-methylpropanesulfonic acid (AMPS) and an N-vinylcarboxamide, or a mixture of cyclic and linear N-vinylcarboxamide, as disclosed in US Patent No. 6,437,068, which is hereby incorporated by reference. A preferred sulfonic acid copolymer thickener is a copolymer of AMPS and N-vinylpyrrolidone.

The pearlescent effect of the present invention appears to be produced by the interaction of the siloxane compound, trimethylsilylalkylsilsesquioxane in the alcoholic hydrogel. It is believed that the trimethylsilylalkylsilsesquioxanes, such as stearoxytrimethylsilane, which is initially a liquid in anhydrous form, after dispersion in the alcoholic hydrogel, undergoes a degree of hydrolysis which results in the formation of finely dispersed crystals of pure stearyl alcohol in the alcoholic hydrogel. This process continues until the pearlescent appearance, or the desired degree of opacity is achieved. The degree of pearlescence or the density of the opacity can be controlled by controlling the amount of the siloxane and other components. The pH of the mixture influences the speed of the hydrolysis reaction. Preferably, the pH of the mixture of the alcoholic hydrogel and the trimethylsilylalkylsilsesquioxane is from 3 to 6. It was discovered that stearoxytrimethylsilane quickly hydrolyses below a pH of 6.

It is preferred that the pearlescent alcoholic hydrogel compositions of the present comprise alcohol from about 2.5 to about 80 weight percent based on the weight of the total alcoholic hydrogel composition. More preferably, the pearlescent alcoholic hydrogel compositions of the present comprise alcohol from about 20 to about 75 weight percent based on the total weight of the alcoholic hydrogel composition, and most preferably, the pearlescent alcoholic hydrogel compositions of the present comprise alcohol from about 40 to about 70 weight percent based on the total weight of the alcoholic hydrogel composition.

It is preferred that the stearoxytrimethylsilane comprises from about 0.01 to about 10 weight percent of the alcoholic hydrogel based on the total weight of the alcoholic hydrogel composition. More preferably, the stearoxytrimethylsilane comprises from about 0.25 to about 8 weight percent of the alcoholic hydrogel based on the total composition, and most preferably, the stearoxytrimethylsilane comprises from about 0.5 to about 2 weight percent based on the total weight of the alcoholic hydrogel composition. In general, when the pearlescent alcoholic hydrogel composition contained more than about 6 weight percent stearoxytrimethylsilane, the pearlescent effect appeared grainy which is aesthetically less preferred. In addition, the greater concentration of stearoxytrimethylsilane in the alcoholic hydrogel composition, the more opaque was the appearance of the hydrogel.

It is preferred that the thickener comprises from about 0.25 to about 5 weight percent based on the total weight of the alcoholic hydrogel alcoholic composition. More preferably, the thickener comprises from about 0.5 to about 5 weight percent based on the weight of the weight of the total alcoholic hydrogel composition, and most preferably, the thickener comprises from about 0.5 to about 2 weight percent based on the weight of the total alcoholic hydrogel composition. The amount of thickener used in the alcoholic hydrogel is dependent upon the type of thickener used and the other components in the hydrogel. In general, the greater the percentage of alcohol, higher the level of thickener required to obtain the gel.

### Optional Ingredient

In addition to alcohol, water and thickener system, the compositions of the present invention may optionally include ingredients such as emollients, antimicrobials, preservatives, dyes, fragrances and therapeutic agents. Each of these optional ingredients is discussed below.

Emollients are typically added to hand lotions or hand preps because they act to increase the moisture content of the stratum corneum. Emollients are generally separated into two broad classes based on their function. The first class of emollients function by forming an occlusive barrier to prevent water evaporation from the stratum corneum. The second class of emollients penetrate into the stratum corneum and physically bind water to prevent evaporation. The first class of emollients is subdivided into compounds which are waxes at room temperature and compounds which are liquid oils. The second class of emollients includes those which are water soluble and are often referred to as humectants.

Suitable preservatives are, for example, phenoxyethanol, formaldehyde solution, parabens, pentanediol or sorbic acid.

Dyes which may be used are the substances approved and suitable for cosmetic purposes and which are compatible with the other components.

In addition to the lower alcohols present in the composition of the present invention, other antimicrobials may be added to enhance the antimicrobial action of the compositions of the present invention. This may be particularly desirable in critical uses such as presurgical hand scrubs or presurgical patient skin scrub replacements. Typical antimicrobials include: iodine and its complexed forms such as povidone/iodine, chlorhexidine salts, such as chlorhexidine digluconate (CHG), parachlorometaxylenol (PCMX), hexachlorophene, phenols, surfactants comprising a long chain hydrophobe (C12-C22) and a quaternary group, triclosan, Lauricidin, hydrogen peroxide, silver, silver salts such as silver chloride, silver oxide and silver sulfadiazine, and the like.

The total amount of auxiliaries and additives can be 1 to 10% by weight, preferably 2 to 5% by weight, based on the composition.

### Examples

The invention is illustrated by the following non-limiting examples:

### Example I - Degree of Hydrolysis Variation with pH

The degree of hydrolysis of stearoxytrimethylsilane was determined in an ethanol/water mixture at varying pH levels ranging from 4.4 to 9.1. A mixture comprising 5 weight percent anhydrous stearoxytrimethylsilane (Available from Clariant Corp., as SILCARE 1 M71), 90 weight percent ethanol and 5 weight percent de-ionized water (D.I.) was subjected to various pH levels and the degree of hydrolysis to stearyl alcohol was determined by GC analysis. The results of the degree of hydrolysis of the stearoxytrimethylsilane to stearyl alcohol at 25°C is shown in Table 1.

**Table 1 % Hydrolysis of Stearoxytrimethylsilane to Stearyl Alcohol**

| pH (Initial) | 15 minutes (Initial) | 2 Days | 14 Days |
|---|---|---|---|
| 4.4 | >99 | >99 | >99 |
| 5.3 | >99 | >99 | >99 |
| 5.5 | 55 | 97 | >99 |
| 6.2 | 6 | 36 | 95 |
| 8.1 | 2 | 10 | 48 |
| 9.1 | 2 | 4 | 21 |

A typical pearlescent instant hand sanitizer composition illustrative of this invention is given in the following examples.

### EXAMPLE II - Hand Sanitizer Preparation

| Constituent | Weight Percent |
|---|---|
| SilCare Silicone 1 M71 (Stearoxytrimethylsilane)¹ | 3.50 |
| D.I. Water | 25.33 |
| SDA 40-2 (SDA - ethanol) Aristoflex AVC (Ammonium | 62.00 |
| Acryloyldimethyltaurate/VP Copolymer)¹ | 1.00 |
| Propylene Glycol | 3.00 |
| Glycereth-7 Triacetate | 3.00 |
| Polyglykol 3350 (PEG-75)¹ | 0.50 |
| Genapol G-260 (Glycereth-26)¹ | 0.45 |
| Dow Corning 193 Surfactant (PEG-12 Dimethicone)² | 0.50 |
| Fragrance | 0.35 |
| Blue 1 (0.1 % aq.) | 0.37 |

| | |
|---|---|
| ⁽¹⁾ Clariant Corporation ⁽²⁾ Dow Corning | |

The instant hand sanitizer of Example II was prepared as follows (trade names as defined above are used for simplicity). All ingredients were combined, except the Aristoflex AVC and the SilCare Silicone 1M71, and the combination was mixed until uniform. Then the Aristoflex AVC was added and the mixing was continued until the mixture was uniform. Then the SilCare Silicone 1 M71 was added and the mixing continued until the mixture was uniform. The pH of the mixture was adjusted to a value less than 6, as required, with citric acid. The resulting mixture was allowed to hydrolyze to produce an opaque (non-transparent) pearlized, viscous gel which exhibited a pleasant feeling on the skin.

### EXAMPLE III - Hand Sanitizer Preparation

| Constituent | Weight Percent |
|---|---|
| SilCare Silicone 1 M71 (Stearoxytrimethylsilane)¹ | 3.50 |
| D.I. Water | 33.50 |
| SDA 40-2 (SDA - ethanol) Aristoflex AVC (Ammonium | 62.00 |
| Acryloyldimethyltaurate/VP Copolymer)¹ | 1.00 |

| | |
|---|---|
| ⁽¹⁾ Clariant Corporation | |

The instant hand sanitizer of Example III was prepared in the same manner as the hand sanitizer of Example II. The resulting alcoholic hydrogel was transparent with a light pearlized appearance.

### EXAMPLE IV Hand Sanitizer Preparation - Carbomer

| Constituent | Weight Percent |
|---|---|
| Carbopol Ultrez 10 (Carbomer)¹ | 1.00 |
| D.I. Water | 35.45 |
| SDA 40-2 (SDA - ethanol) | 62.00 |
| Aminomethylpropanol 99% | 0.05 |
| SilCare Silicone 1M71² | 1.50 |

| | |
|---|---|
| ⁽¹⁾ Noveon ⁽²⁾ Clariant Corporation | |

The alcoholic hydrogel of Example IV was prepared with a carbomer thickener in the following manner. (Trade names as defined above are used for simplicity): The water and the ethanol, SDA 40-2, were combined and mixed. The carbomer, Carbopol was slowly sprinkled into the mixture and the mixing was continued until a uniform mixture was obtained. The mixture was then neutralized to a pH of less than 6 with the addition of the Aminomethylpropanol. Then the SilCare Silicone 1 M71 was added and the mixing continued until again the mixture was uniform. The resulting alcoholic hydrogel was an opaque pearlescent gel, had a pH of 4.85 and had a light, powdery appearance. The application to human skin produced a quick-dry feeling.

### EXAMPLE V Hand Sanitizer Preparation - Carbomer

| | |
|---|---|
| Constituent | Weight Percent |
| Carbopol 940 (Carbomer)¹ | 1.00 |
| D.I. Water | 35.43 |
| SDA 40-2 (SDA - ethanol) | 62.00 |
| Aminomethylpropanol 99% | 0.07 |
| SilCare Silicone 1M71² | 1.50 |

| | |
|---|---|
| ⁽¹⁾ Noveon ⁽²⁾ Clariant Corporation | |

The alcoholic hydrogel of Example V prepared in the same manner as Example VI. The resulting alcoholic hydrogel was a semi-translucent, pearlescent gel having a pH of 5.1 and producing a light, powdery, quick-dry feeling on human skin.

| Trade Name | Company | Description |
|---|---|---|
| SilCare Silicone 1 M71 | Clariant Corp. | Stearoxytrimethylsilane |
| Aristoflex AVC | Clariant Corp. | Ammonium Acryloyldimethyl-taurate/vinylpyrrolidone Copolymer |
| SDA 40-2 | Aaper Alcohol | Specially Denatured Ethanol |
| Polyglykol 3350 | Clariant Corp. | Polyethylene glycol, MW |
| Glycereth-7 Triacetate | | Triester of Acetic Acid and Ethoxylated Glycerin |
| Genapol G-260 | Dow Chemical | Ethoxylated Glycerin |
| Carbopol 940 | Noveon | Crosslinked Acrylic Acid Polymer |
| Dow Corning 193 Surfactant | Dow Corning | Ethoxylated Dimethicone |

## Claims

1. A pearlescent alcoholic hydrogel composition, said composition comprising an alcoholic hydrogel, stearoxytrimethylsilane, a thickener and water, wherein the pearlescent composition is essentially free of a pearlescent wax.

2. The pearlescent alcoholic hydrogel composition of claim 1, further comprising at least one additional ingredient selected from the group consisting of an emollient, an antimicrobial, a preservative, a dye, a fragrance, a therapeutic agent, and mixtures thereof.

3. The pearlescent alcoholic hydrogel composition of claim 1, wherein the alcoholic hydrogel comprises from 2.5 to 80 weight percent of a monohydric alcohol having from 1 to 4 carbon atoms per molecule.

4. The pearlescent alcoholic hydrogel composition of claim 1, wherein the alcoholic hydrogel comprises from 20 to 75 weight percent of a monohydric alcohol having from 1 to 4 carbon atoms per molecule.

5. The pearlescent alcoholic hydrogel composition of claim 1, wherein the alcoholic hydrogel comprises from 40 to 70 weight percent of a monohydric alcohol having from 1 to 4 carbon atoms per molecule.

6. The pearlescent alcoholic hydrogel composition of claim 1, wherein the alcoholic hydrogel comprises from 0.01 to 10 weight percent of stearoxytrimethylsilane.

7. The pearlescent alcoholic hydrogel composition of claim 1, wherein the alcoholic hydrogel comprises from 0.25 to 8 weight percent of stearoxytrimethylsilane.

8. The pearlescent alcoholic hydrogel composition of claim 1, wherein the alcoholic hydrogel comprises from 0.5 to 6 weight percent of stearoxytrimethylsilane.

9. The pearlescent alcoholic hydrogel composition of claim 1, wherein the alcoholic hydrogel comprises from 0.25 to 5 weight percent of the thickener.

10. The pearlescent alcoholic hydrogel composition of claim 1, wherein the thickener is selected from the group consisting of a carbomer, a crosslinked sulfonic acid copolymer, and mixtures thereof.

11. The pearlescent alcoholic hydrogel composition of claim 1, wherein the thickener comprises a carbomer and an effective amount of a neutralizing agent.

12. The pearlescent alcoholic hydrogel composition of claim 1, wherein the thickener comprises a crosslinked copolymer of ammonium acryloyldimethyltaurate and N-vinylcarboxamide.

13. The pearlescent alcoholic hydrogel composition of claim 1, wherein the thickener comprises a crosslinked copolymer of ammonium acryloyldimethyltaurate and N-vinylpyrrolidone.

14. The pearlescent alcoholic hydrogel composition of claim 1, wherein the alcoholic hydrogel comprises from 0.5 to 2 weight percent of the thickener.

15. A method for the cold production of a pearlescent alcoholic hydrogel composition, said method comprising
a) forming an alcoholic hydrogel by combining a monoalcohol and an effective amount of thickener;
b) combining anhydrous stearoxytrimethylsilane with the alcoholic hydrogel at a mixing temperature of between 10 and 30°C;
c) adjusting the pH to between 3 and 6; and,
d) hydrolizing the stearoxytrimethylsilane at said mixing temperature to form the pearlescent alcoholic hydrogel composition.

16. The method of claim 15, further comprising adding to the pearlescent alcoholic hydrogel composition at least one additional ingredient selected from the group consisting of an emollient, an antimicrobial, a preservative, a dye, a fragrance, a therapeutic agent, and mixtures thereof.

17. The method of claim 15, wherein the thickener is selected from the group consisting of a carbomer, a crosslinked sulfonic acid copolymer and mixtures thereof.

18. The method of claim 15, wherein the thickener comprises a carbomer and an effective amount of a neutralizing agent.

19. The method of claim 15, wherein the thickener comprises a crosslinked sulfonic acid copolymer.

20. The method of claim 15 wherein the thickener comprises a copolymer of 2-acrylamido-2-methylpropanesulfonic acid (AMPS) and an N-vinylcarboxamide, or a mixture of cyclic and linear N-vinylcarboxamide.

21. A hand sanitizer produced according to the method of claim 15.
